(19)

**Europäisches Patentamt**

**European Patent Office**

**Office européen des brevets**

(11) **EP 4 714 341 A1**

(12) **EUROPEAN PATENT APPLICATION**

(43) Date of publication:
**25.03.2026 Bulletin 2026/13**

(21) Application number: **24201063.5**

(22) Date of filing: **18.09.2024**

(51) International Patent Classification (IPC):
**A61B 5/024** (2006.01)     **A61B 5/11** (2006.01)
**A61B 5/22** (2006.01)     **A61B 5/00** (2006.01)
**G16H 20/30** (2018.01)

(52) Cooperative Patent Classification (CPC):
**A61B 5/222; A61B 5/024; A61B 5/1118;**
**A61B 5/4866; A61B 5/7275; G16H 20/30;**
A61B 2503/10

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicants:
 • **Imec VZW**
  **3001 Leuven (BE)**
 • **Universiteit Antwerpen**
  **2000 Antwerpen (BE)**

(72) Inventors:
 • **VERDONCK, Tim**
  **3001 Leuven (BE)**

 • **LUTIN, Erika**
  **3001 Leuven (BE)**
 • **SERVOTTE, Thomas**
  **3001 Leuven (BE)**
 • **NONNEMAN, Bart**
  **3001 Leuven (BE)**
 • **COLLE, Camille**
  **3001 Leuven (BE)**
 • **KHOLKINE, Leonid**
  **3001 Leuven (BE)**
 • **LATRE, Steven**
  **3001 Leuven (BE)**

(74) Representative: **Ipsilon Belgium**
  **Bellevue 5/501**
  **9050 Gent-Ledeberg (BE)**

(54) **DETERMINING ENDURANCE PERFORMANCE BY A LINEAR MODEL**

(57)  Example embodiments relate to a computer-implemented method for determining endurance performance of a subject based on sensor measurements; the computer-implemented method comprising: obtaining (201) workload data measured by at least one workload sensor, comprising a time series (212) of the workload exerted by the subject during one or more physical activities; obtaining (202) heart rate data measured by at least one heart rate sensor, comprising a time series (211) of the heart rate of the subject during the one or more physical activities; selecting (203) time intervals (213 - 219) within the workload data and the heart rate data based on a set of selection criteria (204); for the respective time intervals (213 - 219), determining (205) an average workload (207) and, for a final portion of the respective time intervals (213 - 219), determining (205) an average heart rate (206); and determining (208) at least a portion of a workload-heart rate relationship (221), indicative for the endurance performance of the subject, by fitting a linear equation (224) to the average workloads and the average heart rates (230) of the respective time intervals (213 - 219).

Fig. 2

EP 4 714 341 A1

## Description

### Field of the Invention

The present invention generally relates to determining the endurance performance of a subject using sensor data, in particular to determining at least a portion of a workload-heart rate relationship of the subject based on heart rate data and workload data.

### Background of the Invention

The endurance performance of endurance athletes is, amongst others, characterized by an aerobic lactate threshold and an anaerobic lactate threshold, also referred to as LT1 and LT2 respectively. The aerobic lactate threshold expresses the minimum workload an endurance athlete has to exert to reach lactate production levels that exceed his/her physiological baseline levels. The anaerobic lactate threshold expresses the workload at which lactate production starts to exceed lactate clearance, thereby resulting in rapid lactate accumulation.

Training plans and structured workouts are typically constructed based on the lactate thresholds of the athlete, in particular on the workload an athlete can exert at the anaerobic threshold. However, accurately determining the lactate thresholds typically requires performing an exercise test at incremental workloads during which blood samples of the athlete are regularly collected. The lactate concentration within the blood samples can then be related to the incremental workloads. These exercise tests have the problem that they are costly, typically require the athlete to perform a maximal effort, have to be performed in a controlled environment, and are generally perceived as unpleasant by most athletes. As such, these tests are rarely performed by recreational athletes and even infrequently performed by professional athletes.

Some methods exist to approximate the anaerobic lactate threshold with a proxy value based on a dedicated test protocol without collecting blood samples. For example, in cycling, a common approach is to perform a ramp-up test where the workload is incrementally increased until failure. This allows determining the power a cyclist can hold for an hour, i.e. the functional threshold power, which can be used as an approximation of the workload at the anaerobic threshold. However, this has the problem that it requires the athlete to perform a maximal effort during a dedicated test, which is undesired as it results in undue fatigue of the athlete thereby interfering with optimal endurance training. This further makes it unsuitable for continuous performance tracking as the negative effects of frequently repeating such maximal efforts are even more undesirable.

Instead of a dedicated test protocol, some methods attempt to approximate the lactate thresholds of an athlete based on data obtained during regular workouts, e.g. by determining a power-duration curve or by analysing the heart rate variability. However, this has the problem that athletes typically do not spend a lot of time in high-intensity zones during regular workouts and therefore still require incorporating regular maximal efforts in their training plan to enable approximation of the anaerobic threshold. Additionally, these approximation methods are typically sensitive to outliers and noise.

It is a further problem that these approximation methods only allow approximating the anaerobic threshold in an individualized manner, and that the aerobic threshold is merely estimated in a non-individualized manner using population-based heuristics.

### Summary of the Invention

It is an object of the present invention, amongst others, to solve or alleviate above identified problems and challenges by determining the endurance performance of a subject based on sensor measurements obtained during regular unstructured workouts without requiring regular maximal efforts, regular high-intensity efforts, or invasive blood sampling.

According to a first aspect, this object is achieved by a computer-implemented method for determining endurance performance of a subject based on sensor measurements. The computer-implemented method comprising:

- obtaining workload data measured by at least one workload sensor, comprising a time series of the workload exerted by the subject during one or more physical activities;
- obtaining heart rate data measured by at least one heart rate sensor, comprising a time series of the heart rate of the subject during the one or more physical activities;
- selecting time intervals within the workload data and the heart rate data based on a set of selection criteria;
- for the respective time intervals, determining an average workload and, for a final portion of the respective time intervals, determining an average heart rate; and
- determining at least a portion of a workload-heart rate relationship, indicative for the endurance performance of the subject, by fitting a linear equation to the average workloads and the average heart rates of the respective time intervals.

The workload data expresses an external effort the subject exerts during the one or more physical activities. The workload data may therefore depend on the performed endurance sport, e.g. power in cycling or rowing, and pace in running or swimming. The workload data may further account for other external effort metrics during an endurance sport, e.g. elevation may be considered in addition to power or pace. The workload data is obtained by at least one workload sensor such as, for example, crank-based power meters in cycling, pedal-based power meters in cycling, a power meter in rowing, or a smart watch configured to measure pace in running or swimming.

The heart rate data expresses an internal effort the subject exerts during the one or more physical activities. In other words, the heart rate data is the cardiovascular response of the subject to the workload of a physical activity. Thus, the workload data and the heart rate data are time series with corresponding time steps. The heart rate data is obtained by at least one heart rate sensor, e.g. a pulse oximeter or photoplethysmography, PPG, sensor provided within a smart watch; or an electrocardiographic sensor provided within a chest strap. The at least one workload sensor and the at least one heart rate sensor may further be provided within a single device, e.g. a smart watch. It is an advantage that the computer-implemented method is agnostic to the used at least one heart rate sensor and the at least one workload sensor.

The workload data and heart rate data are measured during respective one or more physical activities, also referred to as workouts. A plurality of time intervals are selected from the obtained workload data and heart rate data. The time intervals may be intervals spread across the one or more physical activities. The number of time intervals selected from each of the one or more physical activities may, but need not, be equal. For example, five time intervals may be selected from a first physical activity while only one time interval is selected from a second physical activity. The time intervals may have a predetermined fixed length, e.g. 2 minutes. Time intervals are selected based on a set of selection criteria, thereby limiting the amount of heart rate data and workload data to segments that are suitable for determining the workload-heart rate relationship. This has the advantage that it reduces the computational load and, thus, improves the efficiency of determining the workload-heart rate relationship.

For the respective time intervals, a workload-heart rate pair is then obtained comprising an average workload and an average heart rate. The average workloads of the respective workload-heart rate pairs are obtained by determining the average workload during the entire respective time intervals. The average heart rates of the respective workload-heart rate pairs are obtained by determining the average heart rate during a final portion of the respective time intervals. This final portion refers to a trailing portion or ending portion of the time interval where the heart rate has substantially stabilized, i.e. where the heart rate is substantially free of delayed physiological responses such as cardiac lag or heart rate lag. The final portion may, for example, be the second half of the time interval.

In doing so, a set of workload-heart rate pairs is obtained. A linear equation is then fitted to this set of workload-heart rate pairs to determine or approximate at least a portion of a workload-heart rate relationship. A workload-heart rate relationship expresses the relationship between the workload exerted by a subject during a physical activity and the resulting heart rate of the subject. The workload-heart rate relationship is a good indicator of the endurance performance of the subject, as the workload-heart rate relationship can provide insight into the efficiency, endurance, fatigue resistance, training adaptations, and lactate thresholds of the subject. By fitting the linear equation to the workload-heart rate pairs, the typically substantially linear portion of the workload-heart rate relationship between the aerobic threshold and the anaerobic threshold can be approximated.

The computer-implemented method thus allows to determine the endurance performance of a subject without interfering with endurance training, as performing dedicated maximal efforts and/or high-intensity efforts is unnecessary. This has the further advantage that it makes the computer-implemented method suitable for continuous performance tracking. It is a further advantage that the performance of the subject can be determined and tracked based on regular physical activities performed in real-world environments, i.e. without structured training sessions or without performing dedicated test protocols in a controlled lab environment.

According to an example embodiment, the set of selection criteria may comprise at least one of a maximum variation in the workload data; a maximum workload imbalance; a maximum altitude during the time interval; an increase in average heart rate relative to a preceding interval; a minimum energy expenditure; a maximum energy expenditure; a minimum average heart rate; a maximum average heart rate; a maximum rate of change in heart rate; and a maximum rate of change in workload.

According to an example embodiment, the workload data and the heart rate data may comprise a history of workload data and heart rate data measured during a predetermined number of previous physical activities or measured during previous physical activities performed within a predetermined preceding period.

In other words, the one or more physical activities containing the workload and heart rate data may have been performed by the subject within a historical time window. This historical time window may be defined as a predetermined number of previous physical activities, e.g. the last 15 workouts. Alternatively, the historical time window may be defined as a predetermined period, e.g. the last 28 days. The historical time window may preferably go back far enough in time as to include enough data for fitting the linear equation reliably, without going too far back in time such that changes in the subject's endurance performance remain substantially undetectable within the data.

According to an example embodiment, the computer-implemented method may further comprise updating the workload-heart rate relationship based on workload data and heart rate data measured during a next physical activity.

Thus, the workload-heart rate relationship may initially be determined based on a history of workload and heart rate data measured during the historical window. Thereafter, the determined workload-heart rate relationship may be updated based on workload and heart rate data of a most recent physical activity, e.g. after the subject completes a new workout and saves his/her measurements of the activity to a server. Updating the workload-heart rate relationship may be achieved by selecting time intervals within the most recent physical activity, determining new workload-heart rate pairs for those selected time intervals, and refitting the linear equation to an updated set of workload-heart rate pairs that comprises the historical workload-heart rate pairs in addition to the new workload-heart rate pairs. This allows continuous tracking of the endurance performance without interfering with endurance training.

According to an example embodiment, the computer-implemented method may further comprise discarding at least one time segment from the workload data and the heart rate data that includes one or more outliers within the workload data and/or the heart rate data.

In other words, if a time segment of the workload data and/or a time segment of the heart rate data include one or more outliers, that time segment may be discarded from both the workload time series and the heart rate time series as these time series have corresponding time steps. A time segment may correspond to the entire duration of a physical activity or to a portion of the physical activity. An example of such a time segment may be a segment of the heart rate data with null values or extremely low values indicative of a sensor fault or malfunction. Detecting the one or more outliers may be achieved by any outlier detection technique, e.g. heuristics based on domain knowledge, deep learning models, univariate detection methods, multivariate detection methods, or a combination thereof. Outliers may be detected within the heart rate data by itself, within the workload data by itself, or within a combination of the heart rate and workload data, e.g. when the combination of the heart rate and workload data indicates that the subject exerted a very high workload at an unrealistic low heart rate.

According to an example embodiment, the computer-implemented method may further comprise obtaining an estimated workload of a next physical activity based on the heart rate data measured during the next physical activity and the determined workload-heart rate relationship; and detecting one or more outliers within the workload data by comparing the estimated workload with the workload data measured during the next physical activity.

The heart rate measured during a most recent physical activity may thus be used to predict the workload exerted during that most recent physical activity based on the previously determined workload-heart rate relationship, i.e. based on the fitted linear equation. This predicted workload may then be compared to the measured workload during the physical activity. An outlier may be detected if the difference between the predicted workload and the measured workload, i.e. the prediction error, exceeds a predetermined threshold. The entire physical activity may further be discarded if the average prediction error exceeds a predetermined absolute average threshold.

This can further allow detecting overperformance and underperformance during the most recent physical activity, i.e. when the subject performed particularly good or bad during a workout. Positive average prediction errors may indicate that the subject was able to perform better than expected for the same heart rate, i.e. overperformance. Negative average prediction errors may indicate that the subject performed worse than expected for the same heart rate, i.e. underperformance.

According to an example embodiment, the computer-implemented method may further comprise obtaining an estimated heart rate of a next physical activity based on the workload data measured during the next physical activity and the determined workload-heart rate relationship; and detecting one or more outliers within the heart rate data by comparing the estimated heart rate with the heart rate data measured during the next physical activity.

The workload measured during a most recent physical activity may thus be used to predict the heart rate as a result of exerting said workload during that most recent physical activity based on the previously determined workload-heart rate relationship, i.e. based on the fitted linear equation. This predicted heart rate may then be compared to the measured heart rate during the physical activity. An outlier may be detected if the difference between the predicted heart rate and the measured heart rate, i.e. the prediction error, exceeds a predetermined threshold. The entire physical activity may further be discarded if the average prediction error exceeds a predetermined absolute average threshold.

This can further allow detecting overperformance and underperformance during the most recent physical activity, i.e. when the subject performed particularly good or bad during a workout. Positive average prediction errors may indicate that the subject was able to perform better than expected for the same heart rate, i.e. overperformance. Negative average prediction errors may indicate that the subject performed worse than expected for the same heart rate, i.e. underperformance.

According to an example embodiment, the determined portion of the workload-heart rate relationship may comprise the workload-heart rate relationship between an aerobic threshold and an anaerobic threshold.

According to an example embodiment, the computer-implemented method may further comprise determining the workload of the subject at an aerobic threshold based on the determined workload-heart rate relationship and a predetermined heart rate of the subject at the aerobic threshold.

In other words, the heart rate of the subject at the aerobic threshold should be known. This predetermined heart rate may, for example, be obtained by performing a single lactate test in a lab. Thereafter, the predetermined heart rate at the aerobic threshold may be used to derive the workload at the aerobic threshold from the determined workload-heart rate relationship, i.e. by means of the fitted linear equation. This allows determining the workload at the aerobic threshold without repeating a lactate test and without performing dedicated test protocols in controlled circumstances, i.e. based on regular physical activities. This has the advantage that the workload at the aerobic threshold can be determined reliably and in an individualized manner. It is a further advantage that repeated invasive blood sampling can be avoided.

According to an example embodiment, the computer-implemented method may further comprise determining the workload of the subject at an anaerobic threshold based on the determined workload-heart rate relationship and a predetermined heart rate of the subject at the anaerobic threshold.

In other words, the heart rate of the subject at the anaerobic threshold should be known. This predetermined heart rate may, for example, be obtained by performing a single lactate test in a lab. Thereafter, the predetermined heart rate at the anaerobic threshold may be used to derive the workload at the anaerobic threshold from the determined workload-heart rate relationship, i.e. by means of the fitted linear equation. This allows determining the workload at the anaerobic threshold without repeating a lactate test and without performing dedicated test protocols in controlled circumstances, i.e. based on regular physical activities. This has the advantage that the workload at the anaerobic threshold can be determined reliably without having to perform dedicated maximal efforts. It is a further advantage that repeated invasive blood sampling can be avoided.

According to an example embodiment, the computer-implemented method may comprise determining parameter values of a fitness-fatigue model by minimizing a difference between the workload at the anaerobic threshold estimated by the fitness-fatigue model and the determined workload at the anaerobic threshold.

A fitness-fatigue model is a conceptual framework that describes how an athlete's performance is influenced by two competing factors: fitness and fatigue. A fitness-fatigue model predicts how training, i.e. performing physical activities, affects endurance performance over time by balancing the positive effects of fitness gains with the negative effects of accumulated fatigue. A typical fitness-fatigue model predicts a performance metric based on a relationship defined by parameter values and training load. Training load refers to the amount of stress exerted on a subject during a single physical activity or workout.

A problem with fitness-fatigue models is that the parameter values are typically determined in a non-individualized manner using population-based heuristics or averages, as there is no continuous performance metric available to be used as a target to fit the parameter values. As such, fitness-fatigue models with an individualized fit are rare. Available individualized fitness-fatigue models are impractical as they require a long period of frequent field or lab testing to obtain target performance metrics. Moreover, this frequent testing typically requires the subject to perform dedicated maximal efforts which is undesirable.

By using the workload at the anaerobic threshold determined based on the fitted linear equation, a continuous performance metric target can be obtained without performing frequent maximal efforts, long periods of structured training, frequent field or lab testing, or additional measuring protocols outside regular physical activities or training.

According to an example embodiment, minimizing the difference may be performed by a differential evolution algorithm.

This allows determining the parameter values of the fitness-fatigue model in a stable and reliable manner.

According to an example embodiment, the computer-implemented method may further comprise determining a training load of at least one physical activity based on the workload data and/or the heart rate data of the at least one physical activity, wherein the training load is indicative for stress exerted on the subject by the at least one physical activity; and wherein the fitness-fatigue model determines the performance of a subject based on the parameter values and the training load.

According to an example embodiment, the determined parameter values of the fitness-fatigue model may comprise:

- a first scaling factor indicative for an increase in performance due to the fitness effect of the physical activity;
- a second scaling factor indicative for a decrease in performance due to the fatigue effect of the physical activity;
- a first time factor indicative for a decay of the impact of training load on the increase in performance over time; and
- a second time factor indicative for a decay of the impact of training load on the decrease in performance over time.

According to an example embodiment, determining the parameter values of the fitness-fatigue model further comprises constraining the values of the first scaling factor and the second scaling factor between at least 0.01 and at most 3.00, constraining the value of the first time factor between at least 10 days and at most 60 days, and constraining the value of the second time factor between at least 1 day and at most 10 days.

According to an example embodiment, the second scaling factor may be at least equal to the first scaling factor;

and a difference between the first time factor and the second time factor may be at least 10 days.

According to an example embodiment, a performance metric estimated by the fitness fatigue model is defined as

$$P_0 + k_1 \sum_{i=1}^{n-1} w_i e^{-(n-i)/\tau_1} - k_2 \sum_{i=1}^{n-1} w_i e^{-(n-i)/\tau_2} \qquad \text{or} \qquad \text{as}$$

$$P_0 + k_1 \frac{\sum_{i=1}^{n-1} w_i e^{-(n-i)/\tau_1}}{\sum_{i=1}^{n-1} e^{-(n-i)/\tau_1}} - k_2 \frac{\sum_{i=1}^{n-1} w_i e^{-(n-i)/\tau_2}}{\sum_{i=1}^{n-1} e^{-(n-i)/\tau_2}}, \text{ wherein } P_0 \text{ is indicative for the model offset, } k_1 \text{ is the first}$$

scaling factor, $k_2$ is the second scaling factor, $\tau_1$ is the first time factor, $\tau_2$ is the second time factor, $w_i$ is the training load of physical activity $i$, and n is a unit of time.

According to a second aspect, the object is achieved by a data processing system configured to perform the computer-implemented method according to the first aspect.

According to a third aspect, the object is achieved by a computer program comprising instructions which, when the computer program is executed by a computer, cause the computer to perform the computer-implemented method according to the first aspect.

According to a fourth aspect, the object is achieved by a computer-readable medium comprising instructions which, when executed by a computer, cause the computer to perform the computer-implemented method according to the first aspect.

## Brief Description of the Drawings

Fig. 1 shows an example of a lactate curve indicative for the relationship between the workload exerted by a subject and the blood lactate concentration of the subject during a physical activity;

Fig. 2 shows steps of a computer-implemented method for determining the performance of a subject without interfering with endurance training, according to example embodiments;

Fig. 3 shows further steps of the computer-implemented method, according to example embodiments;

Fig. 4 shows further steps of the computer-implemented method for determining the workload of a subject at the aerobic threshold and/or the anaerobic threshold, according to example embodiments;

Fig. 5 shows steps of a computer-implemented method for obtaining an individualized fitness-fatigue model, according to example embodiments; and

Fig. 6 shows an example embodiment of a suitable computing system for performing steps according to example aspects of the invention.

## Detailed Description of Embodiment(s)

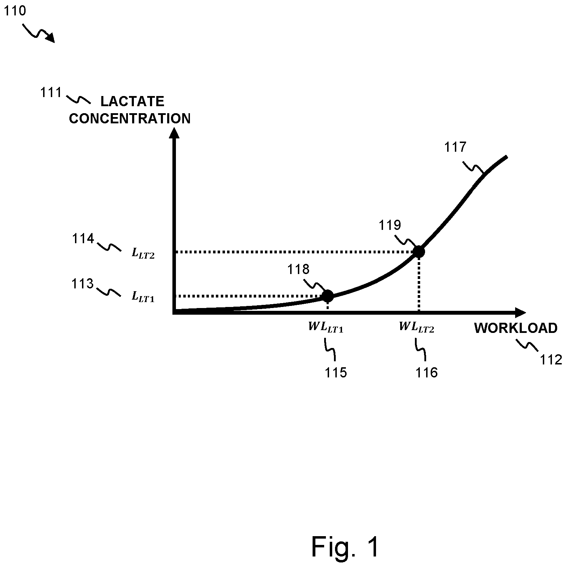

Fig. 1 shows an example of a lactate curve 117 indicative for the relationship between the workload 112 exerted by a subject and the blood lactate concentration 111 of the subject during a physical activity, e.g. cycling, running, swimming, rowing, or cross-country skiing. The lactate curve 117 comprises two points of particular interest for determining the endurance performance of an athlete: the aerobic lactate threshold 118 and the anaerobic lactate threshold 119. The aerobic lactate threshold 118 refers to the minimum workload an endurance athlete has to exert to reach blood lactate production levels that exceed his/her physiological baseline levels. The anaerobic lactate threshold 119 refers to the workload at which blood lactate production starts to exceed lactate clearance, thereby resulting in rapid lactate accumulation within the athlete. The anaerobic lactate threshold 118 typically occurs at a blood lactate concentration 113 of around 2.0 mmol/L, while the anaerobic lactate threshold 119 typically occurs at a blood lactate concentration 114 of around 4.0 mmol/L. However, this can vary between individuals.

The lactate thresholds 118, 119 are typically used to construct training plans and structured workouts for athletes. In particular, the workload 115, 116 an athlete can exert at the respective thresholds 118, 119 is typically used to plan structured training as it is a good indicator of the current endurance performance of the athlete. For example, in cycling, a structured training session may have the athlete perform 4 sets of 15 minute intervals between 70% - 85% of the workload 116 at the anaerobic threshold 119 with 5 minute recoveries between the intervals. As such, accurate and up-to-date knowledge of the lactate thresholds 118, 119 is important as it enables athletes to train optimally by correctly adjusting the intensity of their workouts to their current performance level. However, accurately determining the lactate thresholds 118, 119 typically requires performing an exercise test at incremental workloads during which blood samples of the athlete are regularly collected. This then allows determining the workload 112 the athlete can sustain at certain blood lactate levels 111, thereby obtaining the lactate curve 117

These exercise tests have the problem that they are costly, require the athlete to perform a maximal effort, have to be performed in a controlled environment, and are generally perceived as unpleasant by most athletes. Generally, it is undesirable to perform frequent maximal efforts, as these result in undue accumulation of fatigue and prevent the athlete to train optimally around the moment when the maximal effort has to be performed. In other words, the maximal effort of the exercise test to determine the anaerobic lactate threshold 119 interferes with optimal endurance training. This further makes the blood lactate exercise test unsuitable for continuous performance tracking, as the negative effects of frequently repeating such maximal efforts are even more undesirable. As such, these tests are rarely performed by recreational athletes and even infrequently performed by professional athletes.

Alternatives include approximating the lactate thresholds with a proxy value without collecting blood samples based on dedicated test protocols, e.g. the ramp-up test in cycling, or based on data obtained during regular workouts, e.g. by determining a workload-duration curve or by analysing the heart rate variability.

The approximation methods based on dedicated test protocols have the same problem as the blood lactate test in that they still require a maximal effort during a dedicated test, which interferes with optimal endurance training. The approximation methods based on regular training data have the problem that athletes typically do not spend a lot of time in high-intensity zones, i.e. around the anaerobic threshold 119, during regular workouts and therefore still require incorporating regular maximal efforts in their training plan to enable approximation of the anaerobic threshold 119. Additionally, these approximation methods are typically sensitive to outliers and noise.

It is a further problem that these approximation methods only allow approximating the anaerobic threshold in an individualized manner, and that the aerobic threshold is merely estimated in a non-individualized manner using population-based heuristics.

It can thus be desirable to determine and monitor the performance of a subject without interfering with optimal endurance training, i.e. without performing maximal efforts or high-intensity efforts solely for the purpose of determining the subject's performance.

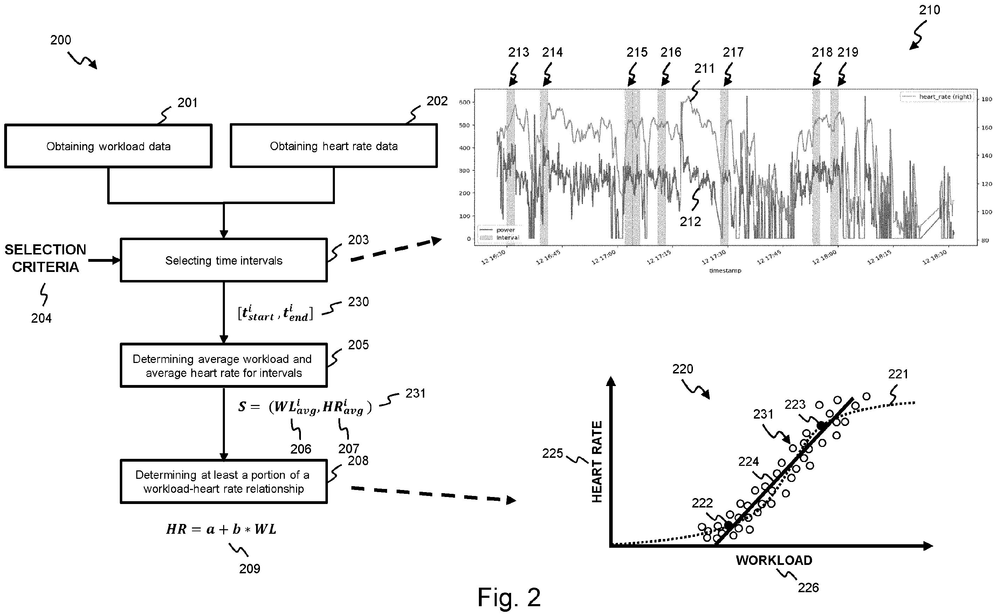

Fig. 2 shows steps 200 of a computer-implemented method for determining the endurance performance of a subject without interfering with endurance training, according to example embodiments.

In a first step 201, 202, workload data and heart rate data are obtained. The workload data and heart rate data are measured during one or more physical activities. The workload data 212 expresses an external effort the subject exerts during the one or more physical activities. The workload data 212 that is measured may therefore depend on the performed endurance sport, e.g. power in cycling or rowing, and pace in running or swimming. The workload data may further account for other external effort metrics during an endurance sport, e.g. elevation may be considered in addition to the pace or power. The workload data is obtained by at least one workload sensor. In cycling, power can for example be measured with a crank-based power meter or a pedal-based power meter. In running or swimming, pace can for example be measured with a smart watch.

The heart rate data 211 expresses an internal effort the subject exerts during the one or more physical activities. The heart rate data thus represents the internal effort of the athlete to exert the external effort of the physical activity. In other words, the heart rate data is the cardiovascular response of the subject to the workload 212 of the physical activity. It will thus be apparent that the workload data 212 and the heart rate data 211 are time series with corresponding time steps, i.e. the data points in the workload data 212 and heart rate data 211 are aligned in time such that each data point in the workload data 212 corresponds with a specific data point in the heart rate data 211. The heart rate data 211 is obtained by at least one heart rate sensor, e.g. a pulse oximeter or photoplethysmography, PPG, sensor provided within a smart watch; or an electrocardiographic sensor provided within a chest strap. The at least one workload sensor and the at least one heart rate sensor may further be provided within a single device, e.g. a smart watch. It is an advantage that the computer-implemented method is agnostic to the at least one heart rate sensor and the at least one workload sensor used.

In a next step 203, time intervals 213 - 219 are selected within the obtained workload data and heart rate data. These time intervals 213 - 219 are selected based on a set of selection criteria 204. The set of selection criteria 204 may comprise a maximum variation in the workload data during the time interval 213 - 219, e.g. the standard deviation of the smoothed workload data may not exceed an absolute threshold of 50W and a relative threshold of 20%. The set of selection criteria 204 may further comprise, a maximum workload imbalance during the time interval 213 - 219 indicative for a maximum difference between the workload exerted by opposing limbs of the subject, e.g. the power imbalance measured at the left and right pedal of a cyclist must be between 0.3 and 0.7. The set of selection criteria 204 may further comprise a maximum altitude during the time interval 213 - 219, e.g. at most 1000 m above sea level. The set of selection criteria 204 may further comprise an increase in average heart rate during the time interval 213 - 219 relative to the average heart rate of a preceding interval. The set of selection criteria 204 may further comprise a minimum energy expenditure and a maximum energy expenditure, e.g. the total energy consumed at the start of the time interval must be between 0kJ and 1500kJ. The set of selection criteria 204 may further comprise a minimum average heart rate; a maximum average heart rate; a maximum rate of change in heart rate; and a maximum rate of change in workload. The set of selection criteria 204 may, for example, comprise that the average heart rate of a final portion of the time segment must be within ($HR_{LT1}$ - $p1$, $HR_{LT2}$ + $p2$) wherein $HR_{LT1}$ is the heart rate of the subject at the aerobic threshold, $HR_{LT2}$ is the heart rate of the subject at the anaerobic threshold, $p1$ is a padding value, and $p2$ is a padding value. The padding values $p1$ and $p2$ may, for example, be 10 and 5 respectively.

By selecting 203 the time intervals 213 - 219 based on the set of selection criteria 204, the amount of heart rate data and workload data can be limited to segments that are suitable for determining the workload-heart rate relationship. This has the advantage that it reduces the computational load and, thus, improves the efficiency of determining the workload-heart rate relationship.

The time intervals 213 - 219 may be defined by a start time $t_{start}^i$ and an end time $t_{end}^i$ within respective physical activities *i*. The time intervals 213 - 219 may have a predetermined fixed length, e.g. 2 minutes. The selected time intervals may be spread across one or more physical activities. The number of time intervals selected from each of the one or more physical activities may, but need not, be equal. For example, five time intervals may be selected from a first physical activity while only one time interval is selected from a second physical activity.

In a following step 205, a workload-heart rate pair is obtained for the respective time intervals 213 - 219. A workload-heart rate pair ( $WL_{avg}^i$, $HR_{avg}^i$ ) comprises an average workload $WL_{avg}^i$ 206 and a corresponding average heart rate $HR_{avg}^i$ 207. The respective average workloads $WL_{avg}^i$ 206 are obtained by determining the average workload during the entire respective time intervals 213 - 219, i.e. during [ $t_{start}^i$, $t_{end}^i$ ]. The average heart rates $HR_{avg}^i$ 207 are obtained by determining the average heart rate during a final portion of the respective time intervals 213 - 219, i.e. during [ $t_{portion}^i$, $t_{end}^i$ ] wherein $t_{start}^i < t_{portion}^i < t_{end}^i$. This final portion thus refers to a trailing portion or ending portion of the time interval where the heart rate has substantially stabilized, i.e. where the heart rate is substantially free of delayed physiological responses such as cardiac lag or heart rate lag. The final portion may, for example, be the second or last half of the time interval. This allows avoiding that the average heart rate, $HR_{avg}^i$ is influenced by the initial portion of the time interval when the heart rate of the subject is still adapting to the workload. In doing so, a set 231 of workload-heart rate pairs is obtained.

In a next step 208, at least a portion of a workload-heart rate relationship 221 is determined by fitting a linear equation 224, 209 to the average workloads and the average heart rates of the respective time intervals 213 - 219, i.e. by fitting a linear equation 224 to the workload-heart rate pairs 231. A workload-heart rate relationship 221 expresses the relationship between the workload 226 exerted by a subject during a physical activity and the resulting heart rate 225 of the subject. The workload-heart rate relationship 221 is a good indicator of the endurance performance of the subject, as the workload-heart rate relationship can provide insight into the efficiency, endurance, fatigue resistance, training adaptations, and lactate thresholds 222, 223 of the subject. By fitting the linear equation 224 to the workload-heart rate pairs 231, the substantially linear portion of a typical workload-heart rate relationship 221 between the aerobic threshold 222 and the anaerobic threshold 223 can be approximated. In other words, the determined portion of the workload-heart rate relationship 221 may preferably comprise the portion of the workload-heart rate relationship between the aerobic threshold 222 and the anaerobic threshold 223.

The computer-implemented method thus allows to determine the endurance performance of a subject without interfering with endurance training, as performing dedicated test protocols with maximal efforts and/or high-intensity efforts is unnecessary. This has the further advantage that it makes the computer-implemented method suitable for continuous performance tracking. It is a further advantage that the performance of the subject can be determined and tracked based on regular unstructured physical activities performed in real-world environments, i.e. without structured training sessions or without performing dedicated test protocols in a controlled lab environment.

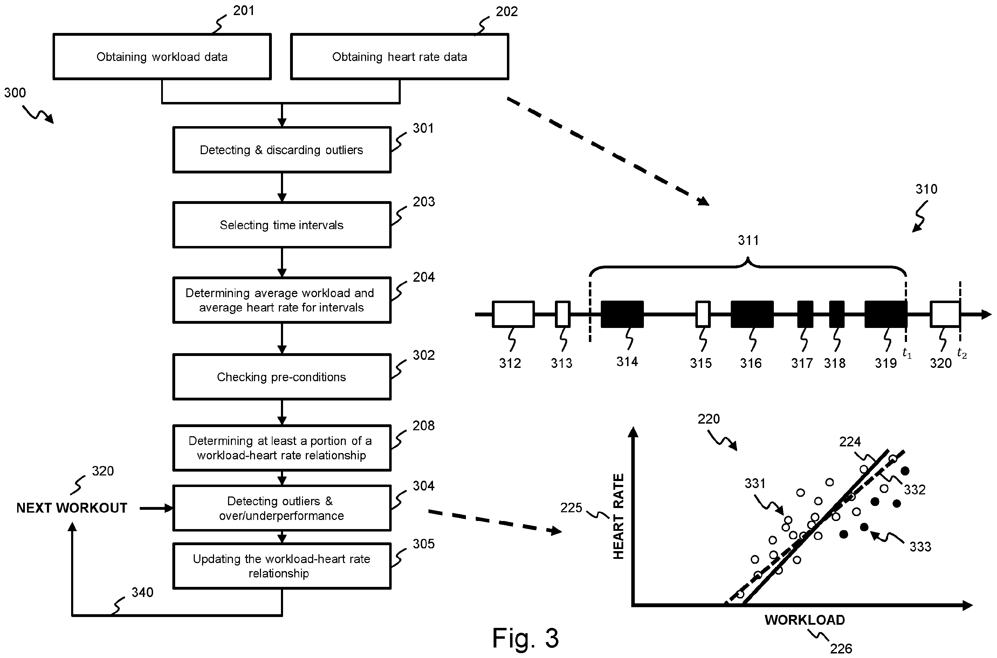

Determining 208 or approximating the portion of the workload-heart rate relationship may initially be performed on a history of workload data and heart rate data obtained during a historical time window. This is illustrated in Fig. 3, which shows a schematic timeline 310 of physical activities 312 - 320 performed by a subject. When the computer-implemented method described in relation to Fig. 2 is executed a first time for a certain subject at timestep $t_1$, the workload data 201 and heart rate data 202 of the physical activities 314 - 319 performed during the historical window 311 may be obtained. This historical time window 311 may be defined as a predetermined number of previous physical activities, e.g. the last 15 workouts. Alternatively, the historical time window 311 may be defined as a predetermined period, e.g. the last 28 days. The historical time window may preferably go back far enough in time as to include enough data for fitting the linear equation 224 reliably, without going too far back in time such that changes in the subject's endurance performance remain substantially undetectable within the data.

Fig. 3 further shows additional steps 301, 302 of the computer-implemented method according to further example embodiments. In step 301, the obtained workload data and heart rate data may be checked for outliers. Outliers can, for example, be null values or extraordinary values caused by faulty sensors, malfunctioning sensors, or environmental factors. Detecting an outlier may be achieved by any outlier detection technique such as, for example, heuristics based on domain knowledge, deep learning models, univariate detection methods, multivariate detection methods, or a combination thereof. If a segment of the workload data and/or the heart rate data comprises one or more outliers, it may be discarded from the data. In other words, if a time segment of the workload data and/or a time segment of the heart rate data include one or more outliers, that time segment may be discarded from both the workload time series and the heart rate time series as these time series have corresponding time steps. Outliers may be detected within the heart rate data by itself, e.g. due to a low battery of the heart rate sensor; within the workload data by itself, e.g. due to a malfunction of the workload sensor; or within a combination of the heart rate and workload data, e.g. when the combination of the heart rate and workload data indicates that the subject exerted a very high workload at an unrealistic low heart rate. It will be apparent that a discarded time segment due to outliers may have any length and, thus, an entire physical activity may be discarded. For example, physical activity 315 may show an excessive number of outliers and may be discarded completely.

From the remaining or retained workload data and heart rate data, e.g. data of physical activities 312, 316, 317, 318, 319 in the example of Fig. 3, time intervals may be selected in step 203 and workload-heart rate pairs may be determined in step 204, as described in relation to Fig. 2. In a next step 302, one or more preconditions for determining the workload-heart rate relationship may be checked. A precondition may be that a minimum number of intervals are selected in step 203, e.g. at least 10 time intervals. Another precondition may be that the selected intervals must belong to a minimum number of unique physical activities 314, 316, 317, 318, 319, e.g. at least 2 distinct physical activities. Another precondition may be that a minimum number of selected intervals have an average heart rate above a certain threshold, e.g. an average heart rate within the upper half of the range $(HR_{LT1} - p1, HR_{LT2} + p2)$ describe in relation to Fig. 2. If the preconditions are met, the computer-implemented method may proceed to step 208 to determine a portion of the workload-heart rate relationship. Otherwise, the computer-implemented method may be interrupted until sufficient high-quality data is available to determine the endurance performance of the subject more reliably.

Thus, after completing step 208, at least a portion of the initial workload-heart rate relationship has been determined by fitting an initial linear equation 224 to the workload-heart rate pairs 331 of previous physical activities 314, 316, 317, 318, 319. The computer-implemented method may further comprise updating 305 this initial workload-heart rate relationship based on workload data and heart rate data measured during a next physical activity 320 or next workout 320. For example, some time after initially determining the endurance performance of the subject at timestep $t_1$, the subject completes a new physical activity 320 at timestep $t_2$. The initial workload-heart rate relationship may then be updated 305 based on this most recent physical activity 320. Updating 305 the workload-heart rate relationship may be achieved by selecting time intervals within the most recent physical activity 320, determining new workload-heart rate pairs 333 for those selected time intervals, and refitting the linear equation 332 to an updated set of workload-heart rate pairs that comprises at least a portion of the historical workload-heart rate pairs 331 in addition to the new workload-heart rate pairs 333. In other words, updating 305 the workload-heart rate relationship may be achieved by at least repeating steps 203, 204, and 205. It will be apparent that the updated set of workload-heart rate pairs may represent a moving window, i.e. the workload-heart rate pairs of the oldest physical activity 314 may be discarded from the set upon adding the new workload-heart rate pairs 333. This allows continuous tracking of the endurance performance without interfering with endurance training.

When a next physical activity 320 becomes available, the computer-implemented method may further also detect outliers within the workload data or heart rate data of that next physical activity 320 based on the initial workload-heart rate relationship, i.e. based on the initial linear equation 224. Detected outliers, or time segments comprising the outliers, may be discarded.

This can be achieved as described in relation to step 301. Alternatively or complementary, the heart rate measured during the most recent physical activity 320 may be used to predict the workload exerted during that activity 320 based on the previously determined workload-heart rate relationship, i.e. based on the initial linear equation 224. This predicted workload may then be compared to the measured workload during the physical activity. An outlier may then be detected if the difference between the predicted workload and the measured workload, i.e. the prediction error, exceeds a predetermined threshold.

Alternatively or complementary, the workload measured during the most recent physical activity 320 may be used to predict the heart rate as a result of exerting said workload during that activity 320 based on the previously determined workload-heart rate relationship, i.e. based on the initial linear equation 224. This predicted heart rate may then be compared to the measured heart rate during the physical activity. An outlier may be detected if the difference between the predicted heart rate and the measured heart rate, i.e. the prediction error, exceeds a predetermined threshold. It will further be apparent that steps 304 and/or 305 may be repeated 340 for each or at least some of the new physical activities 320 that are recorded for a subject.

Comparing the predicted heart rate with the measured heart rate of a most recent physical activity 320 can further allow detecting overperformance and underperformance during the most recent physical activity 320, i.e. when the subject performed particularly good or bad during the most recent workout 320. This can be achieved by selecting time intervals within the most recent physical activity 320, as in step 203, and determining measured workload-heart rate pairs for the selected time intervals. Next, determining predicted workload-heart rate pairs for the selected time intervals by predicting the average heart rates corresponding to the average measured workload for the respective time intervals. This can be achieved by inputting the measured average workloads to the linear equation 224 for the respective time intervals. Thereafter, prediction errors may be determined as the respective differences between the measured workload-heart rate pairs and the predicted workload-heart rate pairs for the respective time intervals. The predictions errors may further be averaged to obtain a single average prediction error for the most recent physical activity 320. A positive average prediction errors may indicate that the subject was able to perform better than expected for the same heart rate, i.e. overperformance. A negative average prediction error may indicate that the subject performed worse than expected for the same heart rate, i.e. underperformance.

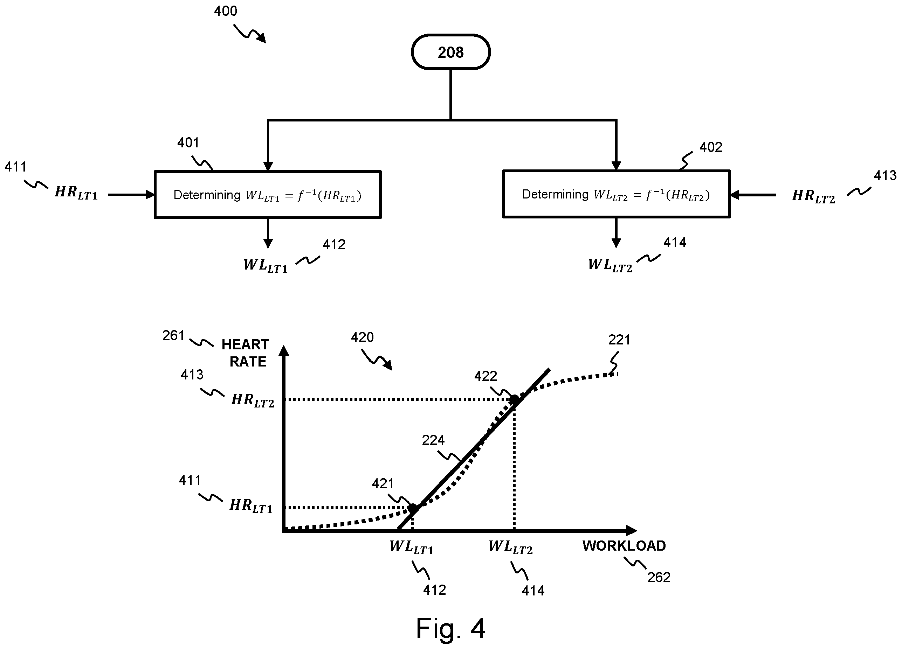

Fig. 4 shows further steps 400 of the computer-implemented method for determining the workload 412, 414 of the subject at the aerobic threshold 421 and/or the anaerobic threshold 422 based on the workload-heart rate relationship approximation, i.e. linear equation 224 $HR = a + b * WL$ determined in step 208. This can be achieved by determining the inverse of the linear equation 224, i.e. $WL = \frac{HR - a}{b}$. This inverse relationship can then be used to determine the workload 412 of the subject at the aerobic threshold 421 in step 401 based on a predetermined heart rate 411 of the subject at the aerobic threshold 421. In other words, the heart rate of the subject at the aerobic threshold should be known.

This predetermined heart rate 411 may, for example, be obtained by performing a single lactate test in a lab. Thereafter, the predetermined heart rate at the aerobic threshold 411 may be used to derive the workload 412 at the aerobic threshold 421 based on the inverse of the fitted linear equation 224. This allows determining the workload 412 at the aerobic threshold 421 without repeating a lactate test and without performing dedicated test protocols in controlled circumstances, i.e. based on regular physical activities. This has the further advantage that the workload at the aerobic threshold can be determined reliably and in an individualized manner.

Alternatively or complementary, the inverse linear equation can be used to determine the workload 414 of the subject at the anaerobic threshold 422 in step 402, based on a predetermined heart rate 413 of the subject at the anaerobic threshold 422. In other words, the heart rate of the subject at the anaerobic threshold should be known. This predetermined heart rate 413 may, for example, be obtained by performing a single lactate test in a lab. Thereafter, the predetermined heart rate at the anaerobic threshold may be used to derive the workload 414 at the anaerobic threshold based on the inverse of the fitted linear equation 224. This allows determining the workload 414 at the anaerobic threshold 422 without repeating a lactate test and without performing dedicated test protocols in controlled circumstances, i.e. based on regular physical activities. This has the advantage that the workload at the anaerobic threshold can be determined reliably without having to perform maximal efforts.

The computer-implemented method thus allows continuously tracking the endurance performance of a subject by periodically determining the workload at the aerobic and/or anaerobic threshold without interfering with endurance training, as frequently performing dedicated maximal and/or high-intensity efforts is unnecessary. The determined workload at the anaerobic threshold can further be used as a continuous performance metric target for fitting an individualized fitness-fatigue model.

A fitness-fatigue model is a conceptual framework that describes how an athlete's performance is influenced by two competing factors: fitness and fatigue. A fitness-fatigue model predicts how training, i.e. performing physical activities, affects endurance performance over time by balancing the positive effects of fitness gains with the negative effects of accumulated fatigue. A typical fitness-fatigue model predicts a performance metric, e.g. the workload at the anaerobic threshold, based on a relationship defined by parameter values and training load. Training load refers to the amount of stress exerted on a subject during a single physical activity or workout.

A problem with fitness-fatigue models is that the parameter values are typically determined in a non-individualized manner using population-based heuristics or averages, as there are no methods to continuously track a performance metric to be used as a target to fit the parameter values without unduly interfering with the training of the subject. As such, fitness-fatigue models with an individualized fit are rare. Available individualized fitness-fatigue models are impractical as they require a long period of frequent field or lab testing to obtain a target performance metric. Moreover, this frequent testing typically requires the subject to perform a maximal effort which is undesirable.

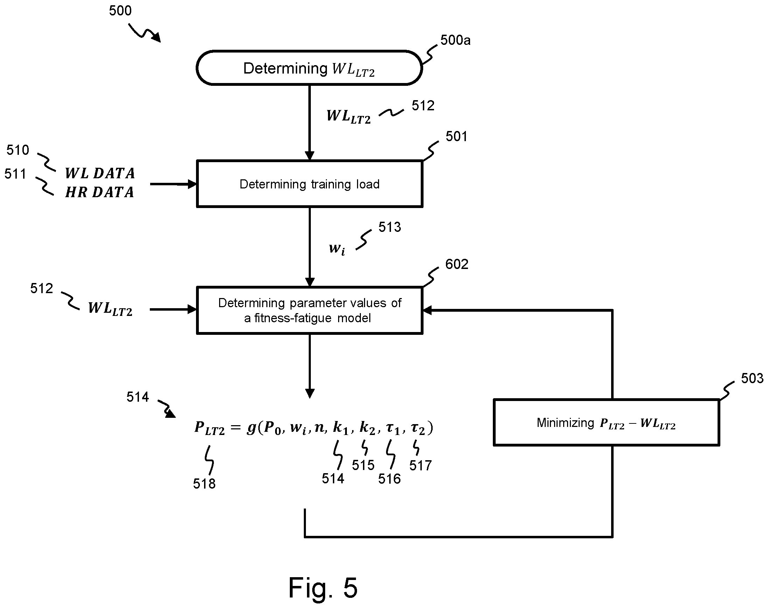

Fig. 5 shows steps 500 of a computer-implemented method for obtaining an individualized fitness-fatigue model according to example embodiments. To this end, the workload at the anaerobic threshold determined as described in relation to Fig. 4 may be obtained in step 500a. In a next step 501, the training load $w_i$ 513 of at least one physical activity may be determined based on workload data 510 and heart rate data 511 measured during the at least one physical activity. Preferably, the training load 513 is determined based on internal load techniques that capture the psychophysiological impact of the workload through subjective scores and heart rate, e.g. Banister's training impulse, bTRIMP, score. However, the training load 513 may also be determined based on external load techniques that quantify the objective workload, e.g. training stress score, TSS.

In the following steps 502 the parameter values of a fitness-fatigue model 514 may be determined. The fitness-fatigue model may be an equation that predicts the workload of a subject at the anaerobic threshold $P_{LT2}$ based on an initial base performance $P_0$, the training load $w_i$ of one or more physical activities $i$, a unit of time n, and parameter values 514 - 517. The parameter values 514 - 517 may preferably comprise a first scaling factor $k_1$ 514 indicative for an increase in performance due to the fitness effect of the physical activity; a second scaling factor $k_2$ 515 indicative for a decrease in performance due to the fatigue effect of the physical activity; a first time factor $\tau_1$ 516 indicative for a decay of the impact of training load on the increase in performance over time; and a second time factor $\tau_2$ 517 indicative for a decay of the impact of training load on the decrease in performance over time. The used fitness-fatigue model may be defined as

$$P_{LT2} = P_0 + k_1 \sum_{i=1}^{n-1} w_i e^{-(n-i)/\tau_1} - k_2 \sum_{i=1}^{n-1} w_i e^{-(n-i)/\tau_2} \qquad \text{(Eq. 1)}$$

Alternatively, the used fitness-fatigue model may be defined as

$$P_{LT2} = P_0 + k_1 \frac{\sum_{i=1}^{n-1} w_i e^{-(n-i)/\tau_1}}{\sum_{i=1}^{n-1} e^{-(n-i)/\tau_1}} - k_2 \frac{\sum_{i=1}^{n-1} w_i e^{-(n-i)/\tau_2}}{\sum_{i=1}^{n-1} e^{-(n-i)/\tau_2}} \qquad \text{(Eq. 2)}$$

The parameter values of the fitness-fatigue model may then be determined by minimizing 503 the difference between the workload at the anaerobic threshold estimated by the fitness-fatigue model $P_{LT2}$ and the determined workload 512 at the anaerobic threshold $WL_{LT2}$. In other words, the parameter values 514 - 517 may be iteratively adjusted as to minimize the difference between $P_{LT2}$ and $WL_{LT2}$. In doing so, an individualized fitness-fatigue model can be obtained without performing maximal efforts, long periods of structured training, frequent field or lab testing, or additional measuring protocols outside regular physical activities or training.

It will be apparent that steps 500 may be performed regularly as to continuously update the determined workload at the anaerobic threshold $WL_{LT2}$ and the parameter values 514 - 517 of the fitness-fatigue model. This has the advantage that personalized, and properly scaled fitness and fatigue variables can be determined and provided to athletes or coaches. This can offer useful applications in endurance training such as, for example, tracking the fitness/fatigue ratio as an indicator of overtraining.

Minimizing the difference in step 503 may further be performed by a differential evolution algorithm. This allows determining the parameter values 514 - 517 in a stable and reliable manner. Minimizing the difference in step 503 may further be subject to constraints based on physiological domain knowledge to limit the search space for the parameter values 514 - 517, thereby improving the convergence speed. The values of the first scaling factor $k_1$ 514 and the second scaling factor $k_2$ 515 may be constrained between at least 0.01 and at most 3.00, i.e. $k_1$ and $k_2 \in [0.01; 3.00]$. The values for the first time factor $\tau_1$ 516 may be constrained between at least 10 days and at most 60 days, i.e. $\tau_1 \in [10,60]$. The values for the second time factor $\tau_2$ 517 may be constrained between at least 1 day and at most 10 days, i.e. $\tau_2 \in [1, 10]$.

To further improve the identified parameter values 514 - 517 for the individualized fitness-fatigue model, minimizing the difference in step 503 may be subject to further constraints that only allow physiologically relevant combinations of the parameter values 514 - 517. These constraints may include that the second scaling factor $k_2$ 515 may be at least equal to the first scaling factor $k_1$ 514, i.e. $k_2 \geq k_1$; and a difference between the first time factor $\tau_1$ 516 and the second time factor $\tau_2$ 517 may be at least 10 days, i.e. $\tau_1 - \tau_2 \geq 10$.

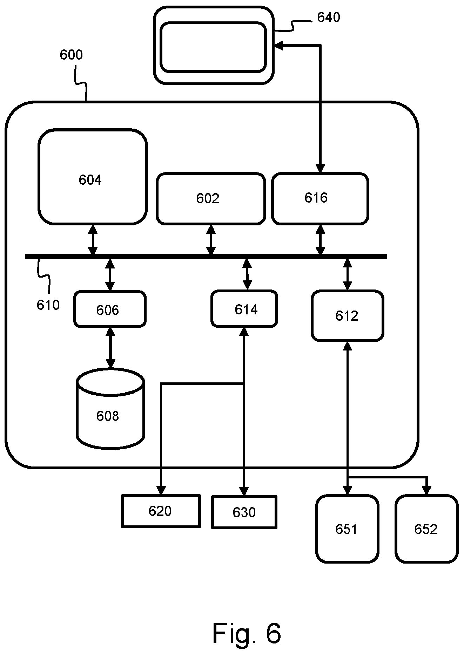

Fig. 6 shows a suitable computing system 600 enabling to implement embodiments of the above described computer-implemented method according to the invention. Computing system 600 may in general be formed as a suitable general-purpose computer and comprise a bus 610, a processor 602, a local memory 604, one or more optional input interfaces 614, one or more optional output interfaces 616, a communication interface 612, a storage element interface 606, and one or more storage elements 608. Bus 610 may comprise one or more conductors that permit communication among the components of the computing system 600. Processor 602 may include any type of conventional processor or microprocessor that interprets and executes programming instructions. Local memory 604 may include a random-access memory (RAM) or another type of dynamic storage device that stores information and instructions for execution by processor 602 and/or a read only memory (ROM) or another type of static storage device that stores static information and instructions for use by processor 602. Input interface 614 may comprise one or more conventional mechanisms that permit an operator or user to input information to the computing device 600, such as a keyboard 620, a mouse 630, a pen, voice recognition and/or biometric mechanisms, a camera, etc. Output interface 716 may comprise one or more conventional mechanisms that output information to the operator or user, such as a display 640, etc. Communication interface 612 may comprise any transceiver-like mechanism such as for example one or more Ethernet interfaces that enables computing system 600 to communicate with other devices and/or systems such as for example, amongst others, at least one heart rate sensor 651 and at least one workload sensor 652. The communication interface 612 of computing system 600 may be connected to such another computing system by means of a local area network (LAN) or a wide area network (WAN) such as for example the internet. Storage element interface 606 may comprise a storage interface such as for example a Serial Advanced Technology Attachment (SATA) interface or a Small Computer System Interface (SCSI) for connecting bus 610 to one or more storage elements 608, such as one or more local disks, for example SATA disk drives, and control the reading and writing of data to and/or from these storage elements 608. Although the storage element(s) 608 above is/are described as a local disk, in general any other suitable computer-readable media such as a removable magnetic disk, optical storage media such as a CD or DVD, -ROM disk, solid state drives, flash memory cards, etc. could be used.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1.   A computer-implemented method for determining endurance performance of a subject based on sensor measurements; the computer-implemented method comprising:

   - obtaining (201) workload data measured by at least one workload sensor, comprising a time series (212) of the workload exerted by the subject during one or more physical activities;
   - obtaining (202) heart rate data measured by at least one heart rate sensor, comprising a time series (211) of the heart rate of the subject during the one or more physical activities;
   - selecting (203) time intervals (213 - 219) within the workload data and the heart rate data based on a set of selection criteria (204);
   - for the respective time intervals (213 - 219), determining (205) an average workload (207) and, for a final portion of the respective time intervals (213 - 219), determining (205) an average heart rate (206); and
   - determining (208) at least a portion of a workload-heart rate relationship (221), indicative for the endurance performance of the subject, by fitting a linear equation (224) to the average workloads and the average heart rates (230) of the respective time intervals (213 - 219).

2.   The computer-implemented method according to claim 1, wherein the set of selection criteria (204) comprises at least one of:

   - a maximum variation in the workload data;
   - a maximum workload imbalance;
   - a maximum altitude during the time interval;
   - an increase in average heart rate relative to a preceding interval;
   - a minimum energy expenditure;
   - a maximum energy expenditure;
   - a minimum average heart rate;
   - a maximum average heart rate;
   - a maximum rate of change in heart rate; and
   - a maximum rate of change in workload.

3. The computer-implemented method according to any of the preceding claims, wherein the workload data (212) and the heart rate data (211) comprise a history of workload data and heart rate data measured during a predetermined number of previous physical activities or measured during previous physical activities performed within a predetermined preceding period.

4. The computer-implemented method according to claim 3, further comprising updating the workload-heart rate relationship (224) based on workload data and heart rate data measured during a next physical activity.

5. The computer-implemented method according to any of the preceding claims, further comprising discarding (301) at least one time segment from the workload data and the heart rate data that includes one or more outliers within the workload data and/or the heart rate data.

6. The computer-implemented method according to claim 5, further comprising obtaining an estimated workload of a next physical activity based on the heart rate data measured during the next physical activity and the determined workload-heart rate relationship; and detecting one or more outliers within the workload data by comparing the estimated workload with the workload data measured during the next physical activity.

7. The computer-implemented method according to claim 5 or 6, further comprising obtaining an estimated heart rate of a next physical activity based on the workload data measured during the next physical activity and the determined workload-heart rate relationship; and detecting one or more outliers within the heart rate data by comparing the estimated heart rate with the heart rate data measured during the next physical activity.

8. The computer-implemented method according to any of the preceding claims, wherein the determined portion of the workload-heart rate relationship comprises the workload-heart rate relationship between an aerobic threshold (222) and an anaerobic threshold (223).

9. The computer-implemented method according to claim 8, further comprising determining (401) the workload (412) of the subject at the aerobic threshold (421) based on the determined workload-heart rate relationship (224) and a predetermined heart rate (411) of the subject at the aerobic threshold (421).

10. The computer-implemented method according to claim 8 or 9, further comprising determining (402) the workload (414) of the subject at the anaerobic threshold (422) based on the determined workload-heart rate relationship (224) and a predetermined heart rate (413) of the subject at the anaerobic threshold (422).

11. The computer-implemented method according to claim 10, further comprising determining (602) parameter values (614, 615, 616, 617) of a fitness-fatigue model (614) by minimizing (603) a difference between the workload at the anaerobic threshold (618) estimated by the fitness-fatigue model and the determined workload at the anaerobic threshold (614).

12. The computer-implemented method according to claim 11, further comprising determining (601) a training load (613) of at least one physical activity based on the workload data (610) and/or the heart rate data (611) of the at least one physical activity, wherein the training load is indicative for stress exerted on the subject by the at least one physical activity; and wherein the fitness-fatigue model (614) determines the performance of a subject based on the parameter values (614, 615, 616, 617) and the training load (613).

13. The computer-implemented method according to any of claims 11 - 12, wherein the determined parameter values of the fitness-fatigue model comprise:

    - a first scaling factor (614) indicative for an increase in performance due to the fitness effect of the physical activity;
    - a second scaling factor (615) indicative for a decrease in performance due to the fatigue effect of the physical activity;
    - a first time factor (616) indicative for a decay of the impact of training load on the increase in performance over time; and
    - a second time factor (617) indicative for a decay of the impact of training load on the decrease in performance over time.

14. The computer-implemented method according to claim 13, wherein determining (602) the parameter values (614, 615, 616, 617) of the fitness-fatigue model (614) further comprises constraining the values of the first scaling factor (614) and the second scaling factor (615) between at least 0.01 and at most 3.00, constraining the value of the first time factor (616) between at least 10 days and at most 60 days, and constraining the value of the second time factor (617) between at least 1 day and at most 10 days.

15. The computer-implemented method according to any of claims 13 - 14, wherein a performance metric estimated by the fitness fatigue model is defined as $P_0 + k_1 \sum_{i=1}^{n-1} w_i e^{-(n-i)/\tau_1} - k_2 \sum_{i=1}^{n-1} w_i e^{-(n-i)/\tau_2}$ or as

$$P_0 + k_1 \frac{\sum_{i=1}^{n-1} w_i e^{-(n-i)/\tau_1}}{\sum_{i=1}^{n-1} e^{-(n-i)/\tau_1}} - k_2 \frac{\sum_{i=1}^{n-1} w_i e^{-(n-i)/\tau_2}}{\sum_{i=1}^{n-1} e^{-(n-i)/\tau_2}}$$

, wherein $P_0$ is indicative for a model offset, $k_1$ is the first scaling factor, $k_2$ is the second scaling factor, $\tau_1$ is the first time factor, $\tau_2$ is the second time factor, $w_i$ is the training load of physical activity $i$, and n is a unit of time.

Fig. 1

EP 4 714 341 A1

200

201
Obtaining workload data

202
Obtaining heart rate data

SELECTION
CRITERIA

204

203
Selecting time intervals

$[t_{start}^{i}, t_{end}^{i}]$ 230

205
Determining average workload and
average heart rate for intervals

$S = (WL_{avg}^{i}, HR_{avg}^{i})$ 231

206   207

208
Determining at least a portion of a
workload-heart rate relationship

$HR = a + b * WL$

209

210

213 214   215 216   211 217   218 219

212

220   221

231   223

225   224

222

HEART RATE

WORKLOAD

226

Fig. 2

16

201

Obtaining workload data

202

Obtaining heart rate data

300

301

Detecting & discarding outliers

203

Selecting time intervals

204

Determining average workload and average heart rate for intervals

302

Checking pre-conditions

208

Determining at least a portion of a workload-heart rate relationship

320

NEXT WORKOUT

304

Detecting outliers & over/underperformance

305

Updating the workload-heart rate relationship

340

310

311

312    313    314    315    316    317    318    319    $t_1$    320    $t_2$

220

224

331

332

225    HEART RATE

333

WORKLOAD

226

Fig. 3

Fig. 4

500

500a

Determining $WL_{LT2}$

$WL_{LT2}$ ~ 512

510
511

**WL DATA**
**HR DATA**

501

Determining training load

$w_i$ 513

512

$WL_{LT2}$

602

Determining parameter values of
a fitness-fatigue model

503

Minimizing $P_{LT2} - WL_{LT2}$

514

$$P_{LT2} = g(P_0, w_i, n, k_1, k_2, \tau_1, \tau_2)$$

518

514  515  516  517

Fig. 5

Fig. 6

Europäisches
Patentamt

European
Patent Office

Office européen
des brevets

# EUROPEAN SEARCH REPORT

**Application Number**

EP 24 20 1063

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/288381 A1 (BARTHOLOMEW JESSE [US] ET AL) 24 November 2011 (2011-11-24) * paragraphs [0005], [0026], [0028], [0031] - [0032], [0040], [0043] - [0045] * | 1-15 | INV. A61B5/024 A61B5/11 A61B5/22 A61B5/00 G16H20/30 |
| X | US 5 976 083 A (RICHARDSON J JEFFREY [US] ET AL) 2 November 1999 (1999-11-02) * column 11, line 35 - column 12, line 48 * * figure 5 * * column 17, line 29 - line 37 * | 1-15 | |
| X | US 2016/058356 A1 (RAGHURAM KARTHIK JAYARAMAN [US] ET AL) 3 March 2016 (2016-03-03) * paragraphs [0023], [0204], [0205] * | 1-15 | |
| X | Mackenzie Brian: "Conconi Test - conduct on a track or treadmill", , 18 July 2024 (2024-07-18), XP093224794, Retrieved from the Internet: URL:https://web.archive.org/web/2024071810 3527/https://www.brianmac.co.uk/coni.htm#g oogle_vignette [retrieved on 2024-11-16] * the whole document * | 1-15 | TECHNICAL FIELDS SEARCHED (IPC) A61B G16H |
| X | US 2020/077949 A1 (BEZZEG PÉTER [HU]) 12 March 2020 (2020-03-12) * paragraphs [0083] - [0084] * | 1-15 | |
| A | EP 3 132 745 B1 (FIRSTBEAT ANALYTICS OY [FI]) 22 December 2021 (2021-12-22) * paragraph [0080] * | 1-15 | |

-/--

The present search report has been drawn up for all claims

| Place of search | Date of completion of the search | Examiner |
|---|---|---|
| The Hague | 23 January 2025 | Papanikolaou, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

# EUROPEAN SEARCH REPORT

**Application Number**

EP 24 20 1063

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KOLOSSA D. ET AL: "Performance Estimation using the Fitness-Fatigue Model with Kalman Filter Feedback", INTERNATIONAL JOURNAL OF COMPUTER SCIENCE IN SPORT, vol. 16, no. 2, 27 November 2017 (2017-11-27), pages 117-129, XP093224822, ISSN: 1684-4769, DOI: 10.1515/ijcss-2017-0010 * section "Methods" * ----- | 1-15 | |

TECHNICAL FIELDS
SEARCHED       (IPC)

The present search report has been drawn up for all claims

| Place of search | Date of completion of the search | Examiner |
|---|---|---|
| The Hague | 23 January 2025 | Papanikolaou, V |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 20 1063

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2025

| Patent document cited in search report | | Publication date | Patent family member(s) | | Publication date |
|---|---|---|---|---|---|
| US 2011288381 | A1 | 24-11-2011 | NONE | | |
| US 5976083 | A | 02-11-1999 | US | 5976083 A | 02-11-1999 |
| | | | US | 6135951 A | 24-10-2000 |
| US 2016058356 | A1 | 03-03-2016 | US | 2016058302 A1 | 03-03-2016 |
| | | | US | 2016058329 A1 | 03-03-2016 |
| | | | US | 2016058332 A1 | 03-03-2016 |
| | | | US | 2016058333 A1 | 03-03-2016 |
| | | | US | 2016058356 A1 | 03-03-2016 |
| | | | US | 2016058370 A1 | 03-03-2016 |
| | | | US | 2016058371 A1 | 03-03-2016 |
| | | | US | 2016058372 A1 | 03-03-2016 |
| US 2020077949 | A1 | 12-03-2020 | EP | 3478157 A1 | 08-05-2019 |
| | | | US | 2020077949 A1 | 12-03-2020 |
| | | | WO | 2018007933 A1 | 11-01-2018 |
| EP 3132745 | B1 | 22-12-2021 | NONE | | |

EPO FORM P0459

For more details about this annex : see Official Journal of the European Patent Office, No. 12/82